# EUROPEAN PATENT SPECIFICATION

(11) **EP 1 987 150 B1**
(45) Date of publication and mention of the grant of the patent: **01.06.2011**
(21) Application number: 07712283.6
(22) Date of filing: 21.02.2007
(51) Int. Cl.: C12N 15/85

(54) **SELECTION OF HOST CELLS EXPRESSING PROTEIN AT HIGH LEVELS**
SELEKTION VON WIRTSZELLEN MIT PROTEINEXPRESSION AUF HOHEM NIVEAU
SÉLECTION DE CELLULES HÔTES EXPRIMANT UNE PROTÉINE À DES NIVEAUX ÉLEVÉS

(30) Priority: 21.02.2006 US 359953; 02.05.2006 US 416490; 02.05.2006 EP 06113354
(43) Date of publication of application: 05.11.2008
(73) Proprietor: ChromaGenics B.V., 2333 CN Leiden (NL)
(72) Inventor: OTTE, Arie, Pieter, NL-3823 SG Amersfoort (NL); VAN BLOKLAND, Henricus Johannes Maria, NL-1456 NH Wijdewormer (NL); KWAKS, Theodorus Hendrikus Jacobus, NL-1022 XW Amsterdam (NL); SEWALT, Richard George Antonius Bernardus, NL-6814 HD Arnhem (NL)
(74) Representative: Verhage, Richard Abraham
(86) International application number: PCT/EP2007/051696
(87) International publication number: WO 2007/096399

(56) References cited:
- WO-A-03/106684
- WO-A-2006/048459
- US-A1- 2006 195 935
- US-B1- 6 319 707
- VAN BLOKLAND ET AL: "A novel, high stringency selection system allows screening of few clones for high protein expression" JOURNAL OF BIOTECHNOLOGY, ELSEVIER SCIENCE PUBLISHERS, AMSTERDAM, NL, vol. 128, no. 2, 13 January 2007 (2007-01-13), pages 237-245, XP005829332 ISSN: 0168-1656
- LOPEZ DE QUINTO S ET AL: "Parameters influencing translational efficiency in aphthovirus IRES-based bicistronic expression vectors" GENE, ELSEVIER, AMSTERDAM, NL, vol. 217, no. 1-2, September 1998 (1998-09), pages 51-56, XP004149318 ISSN: 0378-1119

## Description

### Field of the invention

The invention relates to the field of molecular biology and biotechnology. More specifically the present invention relates to means and methods for improving the selection of host cells that express proteins at high levels.

### Background of the invention

Proteins can be produced in various host cells for a wide range of applications in biology and biotechnology, for instance as biopharmaceuticals. Eukaryotic and particularly mammalian host cells are preferred for this purpose for expression of many proteins, for instance when such proteins have certain posttranslational modifications such as glycosylation. Methods for such production are well established, and generally entail the expression in a host cell of a nucleic acid (also referred to as 'transgene') encoding the protein of interest. In general, the transgene together with a selectable marker gene is introduced into a precursor cell, cells are selected for the expression of the selectable marker gene, and one or more clones that express the protein of interest at high levels are identified, and used for the expression of the protein of interest.

One problem associated with the expression of transgenes is that it is unpredictable, stemming from the high likelihood that the transgene will become inactive due to gene silencing (McBurney et al., 2002), and therefore many host cell clones have to be tested for high expression of the transgene.

Methods to select recombinant host cells that express relatively high levels of desired proteins are known, and several such methods are discussed in the introduction of WO 2006/048459.

In certain advantageous methods in the prior art, bicistronic expression vectors have been described for the rapid and efficient creation of stable mammalian cell lines that express recombinant protein. These vectors contain an internal ribosome entry site (IRES) between the upstream coding sequence for the protein of interest and the downstream coding sequence of the selection marker (Rees et al, 1996). Such vectors are commercially available, for instance the pIRES1 vectors from Clontech (CLONTECHniques, October 1996). Using such vectors for introduction into host cells, selection of sufficient expression of the downstream marker protein then automatically selects for high transcription levels of the multicistronic mRNA, and hence a strongly increased probability of high expression of the protein of interest is envisaged using such vectors. Preferably in such methods, the IRES used is an IRES which gives a relatively low level of translation of the selection marker gene, to further improve the chances of selecting for host cells with a high expression level of the protein of interest by selecting for expression of the selection marker protein (see e.g. WO 03/106684 and WO 2006/005718).

The present invention aims at providing improved means and methods for selection of host cells expressing high levels of proteins of interest.

### Summary of the invention

WO 2006/048459 was filed before but published after the priority date of the instant application, WO 2006/048459 discloses a concept for selecting host cells expressing high levels of polypeptides of interest, the concept referred to therein as 'reciprocal interdependent translation'. In that concept, a multicistronic transcription unit is used wherein a sequence encoding a selectable marker polypeptide is upstream of a sequence encoding a polypeptide of interest, and wherein the translation of the selectable marker polypeptide is impaired by mutations therein, whereas translation of the polypeptide of interest is very high (see e.g. FIG. 13 therein for a schematic view). The present invention provides alternative means and methods for selecting host cells expressing high levels of polypeptide.

In one aspect, the invention provides a. DNA molecule comprising a multicistronic transcription unit coding for i) a polypeptide of interest, and for ii) a selectable marker polypeptide functional in a eukaryotic host cell, wherein the polypeptide of interest has a translation initiation sequence separate from that of the selectable marker polypeptide, and wherein the coding sequence for the polypeptide of interest is upstream from the coding sequence for the selectable marker polypeptide in said multicistronic transcription unit, and wherein an internal ribosome entry site (IRES) is present downstream from the coding sequence for the polypeptide of interest and upstream from the coding sequence for the selectable marker polypeptide, and wherein the nucleic acid sequence coding for the selectable marker polypeptide in the coding strand comprises a translation start sequence chosen from the group consisting of: a) a GTG startcodon; b) a TTG startcodon; c) a CTG startcodon; d) a ATT startcodon; and e) a ACG startcodon.

The translation start sequence in the coding strand for the selectable marker polypeptide comprises a startcodon different from an ATG startcodon, such as one of GTG, TTG, CTG, ATT, or ACG sequence, the first two thereof being the most preferred. Such non-ATG startcodons preferably are flanked by sequences providing for relatively good recognition of the non-ATG sequences as startcodons, such that at least some ribosomes start translation from these startcodons, i.e. the translation start sequence preferably comprises the sequence ACC[non-ATG startcodon]G or GCC[non-ATG startcodon]G.

In preferred embodiments, the selectable marker protein provides resistance against lethal and/or growth-inhibitory effects of a selection agent, such as an antibiotic.

The invention further provides expression cassettes comprising a DNA molecule according to the invention, which expression cassettes further comprise a promoter upstream of the multicistronic expression unit and being functional in a eukaryotic host cell for initiation transcription of the multicistronic expression unit, and said expression cassettes further comprising a transcription termination sequence downstream of the multicistronic expression unit.

In preferred embodiments thereof, such expression cassettes further comprise at least one chromatin control element chosen from the group consisting of a matrix or scaffold attachment region (MAR/SAR), an insulator sequence, a ubiquitous chromatin opener element (UCOE), and an anti-repressor sequence. Anti-repressor sequences are preferred in this aspect, and in certain embodiments said anti-repressor sequences are chosen from the group consisting of: a) any one SEQ. ID. NO. 1 through SEQ. ID. NO. 66; b) fragments of any one of SEQ. ID. NO. 1 through SEQ. ID. NO. 66, wherein said fragments have anti-repressor activity; c) sequences that are at least 70% identical in nucleotide sequence to a) or b) wherein said sequences have anti-repressor activity; and d) the complement to any one of a) to c).

The invention also provides host cells comprising DNA molecules according to the invention.

The invention further provides methods for generating host cells expressing a polypeptide of interest, the method comprising the steps of: introducing into a plurality of precursor host cells a DNA molecule or an expression cassette according to the invention, culturing the cells under conditions selecting for expression of the selectable marker polypeptide, and selecting at least one host cell producing the polypeptide of interest.

In a further aspect, the invention provides methods for producing a polypeptide of interest, the methods comprising culturing a host cell, said host cell comprising an expression cassette according to the invention, and expressing the polypeptide of interest from the expression cassette. In preferred embodiments thereof, the polypeptide of interest is further isolated from the host cells and/or from the host cell culture medium.

### Brief description of the drawings

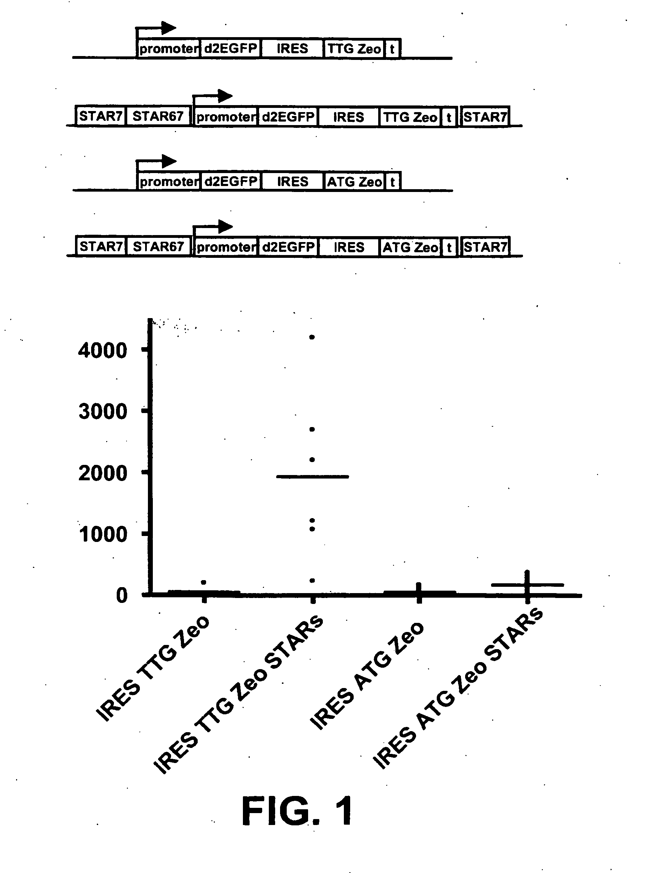

FIG. 1. Results with expression constructs according to the invention. The expression construct contains the sequence encoding the polypeptide of interest (exemplified here by d2EGFP) upstream of an IRES, which is upstream of the sequence encoding the selectable marker according to the invention (exemplified here by the zeocin resistance gene, with a TTG startcodon (TTG Zeo) (or in controls with its normal ATG startcodon (ATG Zeo)). See example 1 for details. Dots indicate individual data points; lines indicate the average expression levels; used constructs are indicated on the horizontal axis, and schematically depicted above the graph; vertical axis indicates d2EGFP signal.

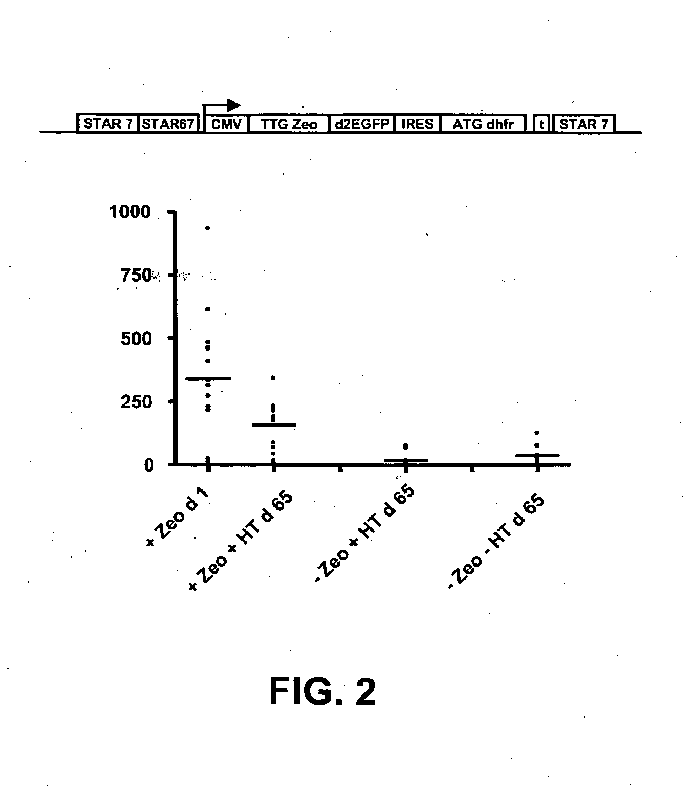

FIG. 2. Results with tricistronic expression cassettes with dhfr as maintenance marker. The expression construct contains a zeocin selectable marker gene with a TTG startcodon and lacking internal ATG sequences upstream of the sequence encoding the polypeptide of interest (exemplified here by d2EGFP), which is further operably linked via an IRES to a downstream metabolic selection marker dhfr gene (with an ATG startcodon). Dots indicate individual data points (GFP fluorescence signal in Zeo^{R} colonies on vertical axis), lines indicate the average expression levels. The used construct is shown above the graph, conditions are indicated on the horizontal axis (d: day). See example 2 for details.

FIG. 3. As Fig 2, but with dhfr gene having GTG startcodon.

FIG. 4. As Fig 2, but with dhfr gene having TTG startcodon.

FIG. 5. Copy numbers in clones with the dhfr enzyme (ATG startcodon), under different conditions. See example 3 for details.

FIG. 6. As Fig. 5, but with dhfr gene having GTG startcodon.

FIG. 7. As Fig. 5, but with dhfr gene having TTG startcodon.

### Detailed description of the invention

In one aspect, the invention provides a DNA molecule according to claim 1. Such a DNA molecule can be used according to the invention for obtaining eukaryotic host cells expressing high levels of the polypeptide of interest, by selecting for the expression of the selectable marker polypeptide. Subsequently or simultaneously, one or more host cell(s) expressing the polypeptide of interest can be identified, and further used for expression of high levels of the polypeptide of interest.

The term "monocistronic gene" is defined as a gene capable of providing a RNA molecule that encodes one polypeptide. A "multicistronic transcription unit", also referred to as multicistronic gene, is defined as a gene capable of providing an RNA molecule that encodes at least two polypeptides. The term "bicistronic gene" is defined as a gene capable of providing a RNA molecule that encodes two polypeptides. A bicistronic gene is therefore encompassed within the definition of a multicistronic gene. A "polypeptide" as used herein comprises at least five amino acids linked by peptide bonds, and can for instance be a protein or a part, such as a subunit, thereof. Mostly, the terms polypeptide and protein are used interchangeably herein. A "gene" or a "transcription unit" as used in the present invention can comprise chromosomal DNA, cDNA, artificial DNA, combinations thereof, and the like. Transcription units comprising several cistrons are transcribed as a single mRNA.

A multicistronic transcription unit according to the invention preferably is a bicistronic transcription unit coding from 5' to 3' for a polypeptide of interest and for a selectable marker polypeptide. Hence, the polypeptide of interest is encoded upstream from the coding sequence for the selectable marker polypeptide. The IRES is operably linked to the sequence encoding the selectable marker polypeptide, and hence the selectable marker polypeptide is dependent from the IRES for its translation.

It is preferred to use separate transcription units for the expression of different polypeptides of interest, also when these form part of a multimeric protein (see e.g. example 13 of WO 2006/048459, the heavy and light chain of an antibody each are encoded by a separate transcription unit, each of these expression units being a bicistronic expression unit).

The DNA molecules of the invention can be present in the form of double stranded DNA, having with respect to the selectable marker polypeptide and the polypeptide of interest a coding strand and a non-coding strand, the coding strand being the strand with the same sequence as the translated RNA, except for the presence of T instead of U. Hence, an AUG startcodon is coded for in the coding strand by an ATG sequence, and the strand containing this ATG sequence corresponding to the AUG startcodon in the RNA is referred to as the coding strand of the DNA. It will be clear to the skilled person that startcodons or translation initiation sequences are in fact present in an RNA molecule, but that these can be considered equally embodied in a DNA molecule coding for such an RNA molecule; hence, wherever the present invention refers to a startcodon or translation initation sequence, the corresponding DNA molecule having the same sequence as the RNA sequence but for the presence of a T instead of a U in the coding strand of said DNA molecule is meant to be included, and vice versa, except where explicitly specified otherwise. In other words, a startcodon is for instance an AUG sequence in RNA, but the corresponding ATG sequence in the coding strand of the DNA is referred to as startcodon as well in the present invention. The same is used for the reference of 'in frame' coding sequences, meaning triplets (3 bases) in the RNA molecule that are translated into an amino acid, but also to be interpreted as the corresponding trinucleotide sequences in the coding strand of the DNA molecule.

The selectable marker polypeptide and the polypeptide of interest encoded by the multicistronic gene each have their own translation initation sequence, and therefore each have their own startcodon (as well as stopcodon), i.e.,they are encoded by separate open reading frames.

The term "selection marker" or "selectable marker" is typically used to refer to a gene and/or protein whose presence can be detected directly or indirectly in a cell, for example a polypeptide that inactivates a selection agent and protects the host cell from the agent's lethal or growth-inhibitory effects (e.g. an antibiotic resistance gene and/or protein). Another possibility is that said selection marker induces fluorescence or a color deposit (e.g. green fluorescent protein (GFP) and derivatives (e.g d2EGFP), luciferase, lacZ, alkaline phosphatase, etc.), which can be used for selecting cells expressing the polypeptide inducing the color deposit, e.g. using a fluorescence activated cell sorter (FACS) for selecting cells that express GFP. Preferably, the selectable marker polypeptide according to the invention provides resistance against lethal and/or growth-inhibitory effects of a selection agent. The selectable marker polypeptide is encoded by the DNA of the invention. The selectable marker polypeptide according to the invention must be functional in a eukaryotic host cell, and hence being capable of being selected for in eukaryotic host cells. Any selectable marker polypeptide fulfilling this criterion can in principle be used according to the present invention. Such selectable marker polypeptides are well known in the art and routinely used when eukaryotic host cell clones are to be obtained, and several examples are provided herein. In certain embodiments, a selection marker used for the invention is zeocin. In other embodiments, blasticidin is used. The person skilled in the art will know that other selection markers are available and can be used, e.g. neomycin, puromycin, bleomycin, hygromycin, etc. In other embodiments, kanamycin is used. In yet other embodiments, the DHFR gene is used as a selectable marker, which can be selected for by methotrexate, especially by increasing the concentration of methotrexate cells can be selected for increased copy numbers of the DHFR gene. The DHFR gene may also be used to complement dhfr-deficiency, e.g. in CHO cells that have a dhfr phenotype, in culture medium with folate and lacking glycine, hypoxanthine and thymidine. Similarly, the glutamine synthetase (GS) gene can be used, for which selection is possible in cells having insufficient GS (e.g. NS-0 cells) by culturing in media without glutamine, or alternatively in cells having sufficient GS (e.g. CHO cells) by adding an inhibitor of GS, methionine sulphoximine (MSX). Other selectable marker genes that could be used, and their selection agents, are for instance described in table 1 of US patent 5,561,053,
; see also Kaufman, Methods in Enzymology, 185:537-566 (1990), for a review of these. If the selectable marker polypeptide is *dhfr,* the host cell in advantageous embodiments is cultured in a culture medium that contains folate and which culture medium is essentially devoid of hypoxanthine and thymidine, and preferably also of glycine.

When two multicistronic transcription units are to be selected for according to the invention in a single host cell, each one preferably contains the coding sequence for a different selectable marker, to allow selection for both multicistronic transcription units. Of course, both multicistronic transcription units may be present on a single nucleic acid molecule or alternatively each one may be present on a separate nucleic acid molecule.

The term "selection" is typically defined as the process of using a selection marker/selectable marker and a selection agent to identify host cells with specific genetic properties (e.g. that the host cell contains a transgene integrated into its genome). It is clear to a person skilled in the art that numerous combinations of selection markers are possible. One antibiotic that is particularly advantageous is zeocin, because the zeocin-resistance protein (zeocin-R) acts by binding the drug and rendering it harmless. Therefore it is easy to titrate the amount of drug that kills cells with low levels of zeocin-R expression, while allowing the high-expressors to survive. All other antibiotic-resistance proteins in common use are enzymes, and thus act catalytically (not 1:1 with the drug). Hence, the antibiotic zeocin is a preferred selection marker. Another preferred selection marker is a 5,6,7,8-tetrahydrofolate synthesizing enzyme (dhfr). However, the invention also works with other selection markers.

A selectable marker polypeptide according to the invention is the protein that is encoded by the nucleic acid of the invention, which polypeptide can be functionally used for selection, for instance because it provides resistance to a selection agent such as an antibiotic. Hence, when an antibiotic is used as a selection agent, the DNA encodes a polypeptide that centers resistance to the selection agent, which polypeptide is the selectable marker polypeptide. DNA sequences coding for such selectable marker polypeptides are known, and several examples of wild-type sequences of DNA encoding selectable marker proteins are provided herein (e.g. Figs. 26-32 of WO 2006/048459). It will be clear that mutants or derivatives of selectable markers can also be suitably used according to the invention, and are therefore included within the scope of the term 'selectable marker polypeptide', as long as the selectable marker protein is still functional.

For convenience and as generally accepted by the skilled person, in many publications as well as herein, often the gene and protein encoding the resistance to a selection agent is referred to as the 'selectable agent (resistance) gene' or 'selection agent (resistance) protein', respectively, although the official names may be different, e.g. the gene coding for the protein conferring restance to neomycin (as well as to G418 and kanamycin) is often referred to as neomycin (resistance) (or neo^{r}) gene, while the official name is aminoglycoside 3'-phosphotransferase gene.

For the present invention, it is beneficial to have low levels of expression of the selectable marker polypeptide, so that stringent selection is possible. In the present invention this is brought about by using a selectable marker coding sequence with a non-ATG startcodon. Upon selection, only cells that have nevertheless sufficient levels of selectable marker polypeptide will be selected, meaning that such cells must have sufficient transcription of the multicistronic transcription unit and sufficient translation of the selectable marker polypeptide, which provides a selection for cells where the multicistronic transcription unit has been integrated or otherwise present in the host cells at a place where expression levels from this transcription unit are high.

The DNA molecules according to the invention have the coding sequence for the selectable marker polypeptide downstream of the coding sequence for the polypeptide of interest. Hence, the multicistronic transcription unit comprises in the 5' to 3' direction (both in the transcribed strand of the DNA and in the resulting transcribed RNA) the sequence encoding the polypeptide of interest and the coding sequence for the selectable marker polypeptide. The IRES is upstream of the coding sequence for the selectable marker polypeptide.

According to the invention, the coding region of the gene of interest is preferably translated from the cap-dependent ORF, and the polypeptide of interest is produced in abundance. The selectable marker polypeptide is translated from an IRES. To decrease translation of the selectable marker cistron, according to the invention the nucleic acid sequence coding for the selectable marker polypeptide comprises a mutation in the startcodon that decreases the translation initiation efficiency of the selectable marker polypeptide in a eukaryotic host cell. Preferably, a GTG startcodon or more prefereably a TTG startcodon is engineered into the selectable marker polypeptide. The translation efficiency .is lower than that of the corresponding wild-type sequence in the same cell, i.e. the mutation results in less polypeptide per cell per time unit, and hence less selectable marker polypeptide.

A translation start sequence is often referred to in the field as 'Kozak sequence', and an optimal Kozak sequence is RCCATGG, the startcodon underlined, R being a purine, i.e. A or G (see Kozak M, 1986, 1987, 1989, 1990, 1997, 2002). Hence, besides the startcodon itself, the context thereof, in particular nucleotides -3 to -1 and +4, are relevant, and an optimal translation startsequence comprises an optimal startcodon (i.e. ATG) in an optimal context (i.e. the ATG directly preceded by RCC and directly followed by G). Translation by the ribosomes is most efficient when an optimal Kozak sequence is present (see Kozak M, 1986, 1987, 1989, 1990, 1997, 2002). However, in a small percentage of events, non-optimal translation initiation sequences are recognized and used by the ribosome to start translation. The present invention makes use of this principle, and allows for decreasing and even fine-tuning of the amount of translation and hence expression of the selectable marker polypeptide, which can therefore be used to increase the stringency of the selection system.

The ATG startcodon of the selectable marker polypeptide in the invention is mutated into another codon, which has been reported to provide some translation initiation, for instance to GTG, TTG, CTG, ATT, or ACG (collectively referred to herein as 'non-ATG start codons'). In preferred embodiments, the ATG startcodon is mutated into a GTG startcodon. This provides still lower expression levels (lower translation) than with the ATG startcodon intact but in a non-optimal context. More preferably, the ATG startcodon is mutated to a TTG startcodon, which provides even lower expression levels of the selectable marker polypeptide than with the GTG startcodon (Kozak M, 1986, 1987, 1989, 1990, 1997, 2002; see also examples 9-13 in WO 2006/048459). The use of non-ATG startcodons in the coding sequence for a selectable marker polypeptide in a multicistronic transcription unit according to the present invention was not disclosed nor suggested in the prior art and, preferably in combination with chromatin control elements, leads to very high levels of expression of the polypeptide of interest, as also shown in WO 2006/048459.

For the use of a non-ATG startcodon according to the invention, it is strongly preferred to provide an optimal context for such a startcodon, i.e. the non-ATG startcodons are preferably directly preceded by nucleotides RCC in positions -3 to -1 and directly followed by a G nucleotide (position +4). However, it has been reported that using the sequence TTTGTGG (startcodon underlined), some initiation is observed at least in vitro, so although strongly preferred it may not be absolutely required to provide an optimal context for the non-ATG startcodons.

ATG sequences within the coding sequence for a polypeptide, but excluding the ATG startcodon, are referred to as `internal ATGs', and if these are in frame with the ORF and therefore code for methionine, the resulting methionine in the polypeptide is referred to as an 'internal methionine'. In the invention of WO 2006/048459 the coding region (following the startcodon, not necessarily including the startcodon) coding for the selectable marker polypeptide is devoid of any ATG sequence in the coding strand of the DNA, up to (but not including) the startcodon of the polypeptide of interest. WO 2006/048459 discloses how to bring this about and how to test the resulting selectable marker polypeptides for functionality. For the present invention, where the selectable marker polypeptide coding sequence is downstream of an IRES and downstream of the coding sequence for the polypeptide of interest, internal ATGs in the sequence encoding the selectable marker polypeptide can remain intact.

Clearly, it is strongly preferred according to the present invention, that the translation start sequence of the polypeptide of interest comprises an optimal translation start sequence, i.e. having the consensus sequence RCCATGG (startcodon underlined). This will result in a very efficient translation of the polypeptide of interest.

By providing the coding sequence of the marker with different mutations leading to several levels of decreased translation efficiency, the stringency of selection can be increased. Fine-tuning of the selection system is thus possible using the multicistronic transcription units according to the invention: for instance using a GTG startcodon for the selection marker polypeptide, only few ribosomes will translate from this startcodon, resulting in low levels of selectable marker protein, and hence a high stringency of selection; using a TTG startcodon even further increases the stringency of selection because even less ribosomes will translate the selectable marker polypeptide from this startcodon.

It is demonstrated in WO 2006/048459, that the multicistronic expression units disclosed therein can be used in a very robust selection system, leading to a very large percentage of clones that express the polypeptide of interest at high levels, as desired. In addition, the expression levels obtained for the polypeptide of interest appear to be significantly higher than those obtained when an even larger number of colonies are screened using selection systems hitherto known.

In addition to a decreased translation initiation efficiency, it could be beneficial to also provide for decreased translation elongation efficiency of the selectable marker polypeptide, e.g. by mutating the coding sequence thereof so that it comprises several non-preferred codons of the host cell, in order to further decrease the translation levels of the marker polypeptide and allow still more stringent selection conditions, if desired. In certain embodiments, besides the mutation(s) that decrease the translation efficiency according to the invention, the selectable marker polypeptide further comprises a mutation that reduces the activity of the selectable marker polypeptide compared to its wild-type counterpart. This may be used to increase the stringency of selection even further. As non-limiting examples, proline at position 9 in the zeocin resistance polypeptide may be mutated, e.g. to Thr or Phe (see e.g. example 14 of WO 2006/048459), and for the neomycin resistance polypeptide, amino acid residue 182 or 261 or both may further be mutated (see e.g. WO 01/32901).

In some embodiments of the invention, a so-called spacer sequence is placed downstream of the sequence encoding the startcodon of the selectable marker polypeptide, which spacer sequence preferably is a sequence in frame with the startcodon and encoding a few amino acids, and that does not contain a secondary structure (Kozak, 1990). Such a spacer sequence can be used to further decrease the translation initiation frequency if a secondary structure is present in the RNA (Kozak, 1990) of the selectable marker polypeptide (e.g. for zeocin, possibly for blasticidin), and hence increase the stringency of the selection system according to the invention (see e.g. example 14 of WO 2006/048459).

It will be clear that any DNA molecules as described but having mutations in the sequence downstream of the first ATG (startcodon) coding for the selectable marker protein can also be used and are thus also encompassed in the invention, as long as the respective encoded selectable marker protein still has activity. For instance any silent mutations that do not alter the encoded protein because of the redundancy of the genetic code are also encompassed. Further mutations that lead to conservative amino acid mutations or to other mutations are also encompassed, as long as the encoded protein still has activity, which may or may not be lower than that of the wild-type protein as encoded by the indicated sequences. In particular, it is preferred that the encoded protein is at least 70%, preferably at least 80%, more preferably at least 90%, still more preferably at least 95% identical to the proteins encoded by the respective indicated sequences (e.g. as provided in SEQ ID NOs. 68-80 of the sequence listing of the present application). Testing for activity of the selectable marker proteins can be done by routine methods.

It is a preferred aspect of the invention to provide an expression cassette comprising the DNA molecule according to the invention, having the multicistronic transcription unit. Such an expression cassette is useful to express sequences of interest, for instance in host cells. An 'expression cassette' as used herein is a nucleic acid sequence comprising at least a promoter functionally linked to a sequence of which expression is desired. Preferably, an expression cassette further contains transcription termination and polyadenylation sequences. Other regulatory sequences such as enhancers may also be included. Hence, the invention provides an expression cassette comprising in the following order: 5'- promoter - multicistronic transcription unit according to the invention, coding for a polypeptide of interest and downstream thereof a selectable marker polypeptide - transcription termination sequence - 3'. The promoter must be capable of functioning in a eukaryotic host cell, i.e. it must be capable of driving transcription of the multicistronic transcription unit. The promoter is thus operably linked to the multicistronic transcription unit. The expression cassette may optionally further contain other elements known in the art, e.g. splice sites to comprise introns, and the like. In some embodiments, an intron is present behind the promoter and before the sequence encoding the polypeptide of interest. An IRES is operably linked to the cistron that contains the selectable marker polypeptide coding sequence. In further embodiments, a sequence coding for a second selectable marker is present in the multicistronic transcription unit (i.e. this is at least a tricistronic transcription unit in these embodiments). In preferred embodiments thereof, said sequence encoding a second selectable marker polypeptide: a) has a translation initiation sequence separate from that of the polypeptide of interest, b) is positioned upstream of said sequence encoding a polypeptide of interest, c) has no ATG sequence in the coding strand following the startcodon of said second selectable marker polypeptide up to the startcodon of the polypeptide of interest, and d) has a non-optimal translation start sequence, e.g. a GTG startcodon or a TTG startcodon. For such embodiments, a preferred selectable marker polypeptide is a 5,6,7,8-tetrahydrofolate synthesizing enzyme (dhfr). This allows for continuous selection of high levels of expression of the polypeptide of interest, as exemplified in example 2.

To obtain expression of nucleic acid sequences encoding protein, it is well known to those skilled in the art that sequences capable of driving such expression, can be functionally linked to the nucleic acid sequences encoding the protein, resulting in recombinant nucleic acid molecules encoding a protein in expressible format. In the present invention, the expression cassette comprises a multicistronic transcription unit. In general, the promoter sequence is placed upstream of the sequences that should be expressed. Much used expression vectors are available in the art, e.g. the pcDNA and pEF vector series of Invitrogen, pMSCV and pTK-Hyg from BD Sciences, pCMV-Script from Stratagene, etc, which can be used to obtain suitable promoters and/or transcription terminator sequences, polyA sequences, and the like.

Where the sequence encoding the polypeptide of interest is properly inserted with reference to sequences governing the transcription and translation of the encoded polypeptide, the resulting expression cassette is useful to produce the polypeptide of interest, referred to as expression. Sequences driving expression may include promoters, enhancers and the like, and combinations thereof. These should be capable of functioning in the host cell, thereby driving expression of the nucleic acid sequences that are functionally linked to them. The person skilled in the art is aware that various promoters can be used to obtain expression of a gene in host cells. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter (Kaufman, 2000). According to the present invention, strong promoters that give high transcription levels in the eukaryotic cells of choice are preferred. Suitable promoters are well known and available to the skilled person, and several are described in WO 2006/048459 (e.g. page 28-29), including the CMV immediate early (IE) promoter (referred to herein as the CMV promoter) (obtainable for instance from pcDNA, Invitrogen), and many others.

In certain embodiments, a DNA molecule according to the invention is part of a vector, e.g. a plasmid. Such vectors can easily be manipulated by methods well known to the person skilled in the art, and can for instance be designed for being capable of replication in prokaryotic and/or eukaryotic cells. In addition, many vectors can directly or in the form of isolated desired fragment therefrom be used for transformation of eukaryotic cells and will integrate in whole or in part into the genome of such cells, resulting in stable host cells comprising the desired nucleic acid in their genome.

Conventional expression systems are DNA molecules in the form of a recombinant plasmid or a recombinant viral genome. The plasmid or the viral genome is introduced into (eukaryotic host) cells and preferably integrated into their genomes by methods known in the art, and several aspects hereof have been described in WO 2006/048459 (e.g. pag. 30-31),

It is widely appreciated that chromatin structure and other epigenetic control mechanisms may influence the expression of transgenes in eukaryotic cells (e.g. Whitelaw et al, 2001). The multicistronic expression units according to the invention form part of a selection system with a rather rigourous selection regime. This generally requires high transcription levels in the host cells of choice. To increase the chance of finding clones of host cells that survive the rigorous selection regime, and possibly to increase the stability of expression in obtained clones, it will generally be preferable to increase the predictability of transcription. Therefore, in preferred embodiments, an expression cassette according to the invention further comprises at least one chromatin control element. A 'chromatin control element' as used herein is a collective term for DNA sequences that may somehow have an effect on the chromatin structure and therewith on the expression level and/or stability of expression of transgenes in their vicinity (they function 'in cis', and hence are placed preferably within 5 kb, more preferably within 2 kb, still more preferably within 1 kb from the transgene) within eukaryotic cells. Such elements have sometimes been used to increase the number of clones having desired levels of transgene expression. Several types of such elements that can be used in accordance with the present invention have been described in WO 2006/048459 (e.g. page 32-34),
, and for the purpose of the present invention chromatin control elements are chosen from the group consisting of matrix or scaffold attachment regions (MARs/SARs), insulators such as the beta-globin insulator element (5' HS4 of the chicken beta-globin locus), scs, scs', and the like, a ubiquitous chromatin opening element (UCOE), and anti-repressor sequences (also referred to as 'STAR' sequences).

Preferably, said chromatin control element is an anti-repressor sequence, preferably chosen from the group consisting of: a) any one SEQ. ID. NO. 1 through SEQ. ID. NO. 66; b) fragments of any one of SEQ. ID. NO. 1 through SEQ. ID. NO. 66, wherein said fragments have anti-repressor activity ('functional fragments'); c) sequences that are at least 70% identical in nucleotide sequence to a) or b) wherein said sequences have anti-repressor activity ('functional derivatives'); and d) the complement to any one of a) to c). Preferably, said chromatin control element is chosen from the group consisting of STAR67 (SEQ. ID. NO. 66), STAR7 (SEQ. ID. NO. 7), STAR9 (SEQ. ID. NO. 9), STAR17 (SEQ. ID. NO. 17), STAR27 (SEQ. ID. NO. 27), STAR29 (SEQ. ID. NO. 29), STAR43 (SEQ. ID. NO. 43), STAR44 (SEQ. ID. NO. 44), STAR45 (SEQ. ID. NO. 45), STAR47 (SEQ. ID. NO. 47), STAR61 (SEQ. ID. NO. 61), or a functional fragment or derivative of said STAR sequences. In a preferred embodiment, said STAR sequence is STAR 67 (SEQ. ID. NO. 66) or a functional fragment or derivative thereof. In certain preferred embodiments, STAR 67 or a functional fragment or derivative thereof is positioned upstream of a promoter driving expression of the multicistronic transcription unit. In other preferred embodiments, the expression cassettes according to the invention are flanked on both sides by at least one anti-repressor sequence, e.g. by one of SEQ. ID. NO. I through SEQ. ID. NO. 65 on both sides, preferably each with the 3' end of these sequences facing the transcription unit. In certain embodiments, expression cassettes are provided according to the invention, comprising in 5' to 3' order: anti-repressor sequence A - anti-repressor sequence B - [promoter - multicistronic transcription unit according to the invention (encoding the polypeptide of interest and downstream thereof the functional selectable marker protein) - transcription termination sequence] - anti-repressor sequence C, wherein A, B and C may be the same or different.

Sequences having anti-repressor activity (anti-repressor sequences) and characteristics thereof, as well as functional fragments or derivatives thereof, and structural and functional definitions thereof, and methods for obtaining and using them, which sequences are useful for the present invention, have been described in WO 2006/048459 (e.g. page 34-38), .

For the production of multimeric proteins, two or more expression cassettes can be used. Preferably, both expression cassettes are multicistronic expression cassettes according to the invention, each coding for a different selectable marker protein, so that selection for both expression cassettes is possible. This embodiment has proven to give good results, e.g. for the expression of the heavy and light chain of antibodies. It will be clear that both expression cassettes may be placed on one nucleic acid molecule or both may be present on a separate nucleic acid molecule, before they are introduced into host cells. An advantage of placing them on one nucleic acid molecule is that the two expression cassettes are present in a single predetermined ratio (e.g. 1:1) when introduced into host cells. On the other hand, when present on two different nucleic acid molecules, this allows the possibility to vary the molar ratio of the two expression cassettes when introducing them into host cells, which may be an advantage if the preferred molar ratio is different from 1:1 or when it is unknown beforehand what is the preferred molar ratio, so that variation thereof and empirically finding the optimum can easily be performed by the skilled person. According to the invention, preferably at least one of the expression cassettes, but more preferably each of them, comprises a chromatin control element, more preferably an anti-repressor sequence.

In another embodiment, the different subunits or parts of a multimeric protein are present on a single expression cassette.

Useful configurations of anti-repressors combined with expression cassettes have been described in WO 2006/048459 (e.g. page 40),

In certain embodiments, transcription units or expression cassettes according to the invention are provided, further comprising a transcription pause (TRAP) sequence, essentially as described on page 40-41 of WO 2006/048459,
One non-limiting example of a TRAP sequence is given in SEQ. ID. NO. 81. Examples of other TRAP sequences, methods to find these, and uses thereof have been described in WO 2004/055215.

DNA molecules comprising multicistronic transcription units and/or expression cassettes according to the present invention can be used for improving expression of nucleic acid, preferably in host cells. The terms "cell"/"host cell" and "cell line"/"host cell line" are respectively typically defined as a cell and homogeneous populations thereof that can be maintained in cell culture by methods known in the art, and that have the ability to express heterologous or Homologous proteins.

Several exemplary host cells that can be used have been described in WO 2006/048459 (e.g. page 41-42), and such cells include for instance mammalian cells, including but not limited to CHO cells, e.g. CHO-K1, CHO-S, CHO-DG44, CHO-DUKXB11, including CHO cells having a dhfr⁻ phenotype, as well as myeloma cells (e.g. Sp2/0, NS0), HEK 293 cells, and PER.C6 cells.

Such eukaryotic host cells can express desired polypeptides, and are often used for that purpose. They can be obtained by introduction of a DNA molecule of the invention, preferably in the form of an expression cassette, into the cells. Preferably, the expression cassette is integrated in the genome of the host cells, which can be in different positions in various host cells, and selection will provide for a clone where the transgene is integrated in a suitable position, leading to a host cell clone with desired properties in terms of expression levels, stability, growth characteristics, and the like. Alternatively the multicistronic transcription unit may be targeted or randomly selected for integration into a chromosomal region that is transcriptionally active, e.g. behind a promoter present in the genome. Selection for cells containing the DNA of the invention can be performed by selecting for the selectable marker polypeptide, using routine methods known by the person skilled in the art. When such a multicistronic transcription unit is integrated behind a promoter in the genome, an expression cassette according to the invention can be generated in situ, i.e. within the genome of the host cells.

Preferably the host cells are from a stable clone that can be selected and propagated according to standard procedures known to the person skilled in the art. A culture of such a clone is capable of producing polypeptide of interest, if the cells comprise the multicistronic transcription unit of the invention.

Introduction of nucleic acid that is to be expressed in a cell, can be done by one of several methods, which as such are known to the person skilled in the art, also dependent on the format of the nucleic acid to be introduced. Said methods include but are not limited to transfection, infection, injection, transformation, and the like. Suitable host cells that express the polypeptide of interest can be obtained by selection.

In preferred embodiments, the DNA molecule comprising the multicistronic transcription unit of the invention, preferably in the form of an expression cassette, is integrated into the genome of the eukaryotic host cell according to the invention. This will provide for stable inheritance of the multicistronic transcription unit.

Selection for the presence of the selectable marker polypeptide, and hence for expression, can be performed during the initial obtaining of the cells. In certain embodiments, selection agent is present in the culture medium at least part of the time during the culturing, either in sufficient concentrations to select for cells expressing the selectable marker polypeptide or in lower concentrations. In preferred embodiments, selection agent is no longer present in the culture medium during the production phase when the polypeptide is expressed.

A polypeptide of interest according to the invention can be any protein, and may be a monomeric protein or a (part of a) multimeric protein. A multimeric protein comprises at least two polypeptide chains. Non-limiting examples of a protein of interest according to the invention are enzymes, hormones, immunoglobulin chains, therapeutic proteins like anti-cancer proteins, blood coagulation proteins such as Factor VIII, multi-functional proteins, such as erythropoietin, diagnostic proteins, or proteins or fragments thereof useful for vaccination purposes, all known to the person skilled in the art.

In certain embodiments, an expression cassette of the invention encodes an immunoglobulin heavy or light chain or an antigen binding part, derivative and/or analogue thereof. In a preferred embodiment a protein expression unit according to the invention is provided, wherein said protein of interest is an immunoglobulin heavy chain. In yet another preferred embodiment a protein expression unit according to the invention is provided, wherein said protein of interest is an immunoglobulin light chain. When these two protein expression units are present within the same (host) cell a multimeric protein and more specifically an immunoglobulin, is assembled. Hence, in certain embodiments, the protein of interest is an immunoglobulin, such as an antibody, which is a multimeric protein. Preferably, such an antibody is a human or humanized antibody. In certain embodiments thereof, it is an IgG, IgA, or IgM antibody. An immunoglobulin may be encoded by the heavy and light chains on different expression cassettes, or on a single expression cassette. Preferably, the heavy and light chain are each present on a separate expression cassette, each having its own promoter (which may be the same or different for the two expression cassettes), each comprising a multicistronic transcription unit according to the invention, the heavy and light chain being the polypeptide of interest, and preferably each coding for a different selectable marker protein, so that selection for both heavy and light chain expression cassette can be performed when the expression cassettes are introduced and/or present in a eukaryotic host cell.

The polypeptide of interest may be from any source, and in certain embodiments is a mammalian protein, an artificial protein (e.g. a fusion protein or mutated protein), and preferably is a human protein.

Obviously, the configurations of the expression cassettes of the present invention may also be used when the ultimate goal is not the production of a polypeptide of interest, but the RNA itself, for instance for producing increased quantities of RNA from an expression cassette, which may be used for purposes of regulating other genes (e.g. RNAi, antisense RNA), gene therapy, in vitro protein production, etc.

In one aspect, the invention provides a method for generating a host cell expressing a polypeptide of interest, the method comprising introducing into a plurality of precursor cells a DNA molecule or an expression cassette according to the invention, culturing the generated cells under selection conditions and selecting at least one host cell producing the polypeptide of interest. Advantages of this novel method are similar to those described for the alternative method disclosed in WO 2006/048459 (e.g. page 46-47),

While clones having relatively low copy numbers of the multicistronic transcription units and high expression levels can be obtained, the selection system of the invention nevertheless can be combined with amplification methods to even further improve expression levels. This can for instance be accomplished by amplification of a co-integrated dhfr gene using methotrexate, for instance by placing dhfr on the same nucleic acid molecule as the multicistronic transcription unit of the invention, or by cotransfection when dhfr is on a separate DNA molecule. The dhfr gene can also be part of a multicistronic expression unit of the invention.

The invention also provides methods for producing one or more polypeptides of interest, the method comprising culturing host cells of the invention.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof Culturing can be done for instance in dishes, roller bottles or in bioreactors, using batch, fed-batch, continuous systems such as perfusion systems, and the like. In order to achieve large scale (continuous) production of recombinant proteins through cell culture it is preferred in the art to have cells capable of growing in suspension, and it is preferred to have cells capable of being cultured in the absence of animal- or human-derived serum or animal- or human-derived serum components.

The conditions for growing or multiplying cells (see e.g. Tissue Culture, Academic Press, Kruse and Paterson, editors (1973)) and the conditions for expression of the recombinant product are known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991).

In a preferred embodiment, the expressed protein is collected (isolated), either from the cells or from the culture medium or from both. It may then be further purified using known methods, e.g. filtration, column chromatography, etc, by methods generally known to the person skilled in the art.

The selection method according to the present invention works in the absence of chromatin control elements, but improved results are obtained when the multicistronic expression units are provided with such elements. The selection method according to the present invention works particularly well when an expression cassette according to the invention, comprising at least one anti-repressor sequence is used. Depending on the selection agent and conditions, the selection can in certain cases be made so stringent, that only very few or even no host cells survive the selection, unless anti-repressor sequences are present. Hence, the combination of the novel selection method and anti-repressor sequences provides a very attractive method to obtain only limited numbers of colonies with a greatly improved chance of high expression of the polypeptide of interest therein, while at the same time the obtained clones comprising the expression cassettes with anti-repressor sequences provide for stable expression of the polypeptide of interest, i.e. they are less prone to silencing or other mechanisms of lowering expression than conventional expression cassettes.

In one aspect the invention provides a multicistronic transcription unit having an alternative configuration compared to the configuration disclosed in WO 2006/048459: in the alternative configuration of the present invention, the sequence coding for the polypeptide of interest is upstream of the sequence coding for the selectable marker polypeptide, and the selectable marker polypeptide is operably linked to a cap-independent translation initiation sequence, preferably an internal ribosome entry site (IRES). Such multicistronic transcription units as such were known (e.g. Rees et al, 1996, WO 03/106684), but had not been combined with a non-ATG startcodon. According to the alternative of the present invention, the startcodon of the selectable marker polypeptide is changed into a non-ATG startcodon, to further decrease the translation initiation rate for the selectable marker. This therefore leads to a desired decreased level of expression of the selectable marker polypeptide, and can result in highly effective selection host cells expressing high levels of the polypeptide of interest, as with the embodiments disclosed in WO 2006/048459. One potential advantage of this alternative aspect of the present invention, compared to the embodiments outlined in WO 2006/048459, is that the coding sequence of the selectable marker polypeptide needs no further modification of internal ATG sequences, because any internal ATG sequences therein can remain intact since they are no longer relevant for translation of further downstream polypeptides. This may be especially advantageous if the coding sequence for the selectable marker polypeptide contains several internal ATG sequences, because the task of changing these and testing the resulting construct for functionality does not have to be performed for the present invention: only mutation of the ATG startcodon suffices in this case. It is shown hereinbelow (example 1) that this alternative provided by the present invention also leads to very good results.

The coding sequence for the selectable marker polypeptide in the DNA molecules of the present invention is under translational control of the IRES, whereas the coding sequence for the protein of interest is preferably translated in a cap-dependent manner. The coding sequence for the polypeptide of interest comprises a stopcodon, so that translation of the first cistron ends upstream of the IRES, which IRES is operably linked to the second cistron.

As will be readily apparent to the skilled person after reading the present disclosure, most parts of these multicistronic expression units can be advantageously varied along the same lines as for the multicistronic expression units having an opposite order of the coding sequences for the polypeptide of interest and the selectable marker polypeptide (i.e. the multicistronic transcription units of WO 2006/048459). For instance, the preferred startcodons for the selectable marker polypeptide, the incorporation into expression cassettes, the host cells, the promoters, the presence of chromatin control elements, etc. can be varied and used in preferred embodiments as described supra. Also the use of these multicistronic expression units and expression cassettes is as described supra. Therefore, this aspect is really an alternative to the means and methods described in WO 2006/048459, with the main difference being that the order of the polypeptides in the multicistronic expression units is reversed, and that an IRES is now required for the translation of the selectable marker polypeptide.

As used herein, an "internal ribosome entry site" or "IRES" refers to an element that promotes direct internal ribosome entry to the initiation codon, such as normally an ATG, but in this invention preferably GTG or TTG, of a cistron (a protein encoding region), thereby leading to the cap-independent translation of the gene. See, e. g., Jackson R J, Howell M T, Kaminski A (1990) Trends Biochem Sci 15 (12): 477-83) and Jackson R J and Kaminski, A. (1995) RNA 1 (10): 985-1000. The present invention encompasses the use of any cap-independent translation initiation sequence, in particular any IRES element that is able to promote direct internal ribosome entry to the initiation codon of a cistron. "Under translational control of an IRES" as used herein means that translation is associated with the IRES and proceeds in a cap-independent manner. As used herein, the term "IRES" encompasses functional variations of IRES sequences as long as the variation is able to promote direct internal ribosome entry to the initiation codon of a cistron. As used herein, "cistron" refers to a polynucleotide sequence, or gene, of a protein, polypeptide, or peptide of interest. "Operably linked" refers to a situation where the components described are in a relationship permitting them to function in their intended manner. Thus, for example, a promoter "operably linked" to a cistron is ligated in such a manner that expression of the cistron is achieved under conditions compatible with the promoter. Similarly, a nucleotide sequence of an IRES operably linked to a cistron is ligated in such a manner that translation of the cistron is achieved under conditions compatible with the IRES.

Internal ribosome binding site (IRES) elements are known from viral and mammalian genes (Martinez-Salas, 1999), and have also been identified in screens of small synthetic oligonucleotides (Venkatesan & Dasgupta, 2001). The IRES from the encephalomyocarditis virus has been analyzed in detail (Mizuguchi et al., 2000). An IRES is an element encoded in DNA that results in a structure in the transcribed RNA at which eukaryotic ribosomes can bind and initiate translation. An IRES permits two or more proteins to be produced from a single RNA molecule (the first protein is translated by ribosomes that bind the RNA at the cap structure of its 5' terminus, (Martinez-Salas, 1999)). Translation of proteins from IRES elements is less efficient than cap-dependent translation: the amount of protein from IRES-dependent open reading frames (ORFs) ranges from less than 20% to 50% of the amount from the first ORF (Mizuguchi et al., 2000). The reduced efficiency of IRES-dependent translation provides an advantage that is exploited by this embodiment of the current invention. Furthermore, mutation of IRES elements can attenuate their activity, and lower the expression from the IRES-dependent ORFs to below 10% of the first ORF (Lopez de Quinto & Martinez-Salas, 1998, Rees et al., 1996). It is therefore clear to a person skilled in the art that changes to the IRES can be made without altering the essence of the function of the IRES (hence, providing a protein translation initiation site with a reduced translation efficiency), resulting in a modified IRES. Use of a modified IRES which is still capable of providing a small percentage of translation (compared to a 5' cap translation) is therefore also included in this invention. The present invention uses non-ATG startcodons to significantly further reduce translation initation of the selectable marker ORF, therewith further improving the chances of obtaining a preferred host cell, i.e. a host cell expressing high levels of recombinant protein of interest.

US patents 5,648,267 and 5,733,779 describe the use of a dominant selectable marker sequence with an impaired consensus Kozak sequence ([Py]xxATG[Py], wherein [Py] is a pyrimidine nucleotide (i.e. C or T), x is a nucleotide (i.e. G, A, T, or C), and the ATG startcodon is underlined). US patent 6,107,477 describes the use of a non-optimal Kozak sequence (AGATCTTTATGGACC, wherein the ATG startcodon is underlined) for a selectable marker gene. None of these patents describes the use of a non-ATG startcodon, nor provides any suggestion to do so. Furthermore they are silent on combinations with an IRES. Moreover, since an IRES in itself already has reduced translation initiation compared to cap-dependent translation, it could not be foreseen prior to the present invention whether the combination of an IRES with a non-ATG startcodon for the selectable marker could provide sufficient translation of the selectable marker polypeptide to give any selectable levels thereof. The present invention shows that this is the case, and provides surprisingly efficient selection systems.

The invention also provides a DNA molecule comprising a sequence coding for a selectable marker polypeptide operably linked to an IRES sequence, wherein the coding sequence coding for the selectable marker polypeptide comprises a translation start sequence selected from the group consisting of: a) a GTG start codon; b) a TTG start codon; c) a CTG start codon; d) a ATT start codon; and e) a ACG start codon.

The skilled person will understand that further modifications of the invention are possible, e.g. those given in US 2006/0195935, particularly examples 20-27 thereof.

In certain embodiments, the mammalian 5,6,7,8 tetrahydrofolate synthesizing enzyme dihydrofolate reductase (*dhfr*) can be used as a selection marker in cells that have a *dhfr* phenotype (e.g. CHO-DG44 cells), by omitting hypoxanthine and thymidine (and preferably also glycine) from the culture medium and including folate (or (dihydro)folic acid) into the culture medium (Simonsen et al, 1988). The *dhfr* gene can for instance be derived from the mouse genome or mouse cDNA and can be used according to the invention, preferably by providing it with a GTG or TTG startcodon (see SEQ. ID. NO. 73 for the sequence of the *dhfr* gene). In all these embodiments, by 'omitting from the culture medium' is meant that the culture medium has to be essentially devoid of the indicated component(s), meaning that there is insufficient of the indicated component present to sustain growth of the cells in the culture medium, so that a good selection is possible when the genetic information for the indicated enzyme is expressed in the cells and the indicated precursor component is present in the culture medium. For instance, the indicated component is present at a concentration of less than 0.1 % of the concentration of that component that is normally used in the culture medium for a certain cell type. Preferably, the indicated component is absent from the culture medium. A culture medium lacking the indicated component can be prepared according to standard methods by the skilled person or can be obtained from commercial media suppliers. A potential advantage of the use of these types of metabolic enzymes as selectable marker polypeptides is that they can be used to keep the multicistronic transcription units under continuous selection, which may result in higher expression of the polypeptide of interest.

In another aspect, the invention uses the *dhfr* metabolic selection marker as an additional selection marker in a multicistronic transcription unit according to the invention. In such embodiments, selection of host cell clones with high expression is first established by use of for instance an antibiotic selection marker, e.g. zeocin, neomycin, etc, the coding sequences of which will have a GTG or TTG startcodon according to the invention. After the selection of suitable clones, the antibiotic selection is discontinued, and now continuous or intermittent selection using the metabolic enzyme selection marker can be performed by culturing the cells in the medium lacking the appropriate identified components described supra and containing the appropriate precursor components described supra. In this aspect, the metabolic selection marker is operably linked to an IRES, and can have its normal ATG content, and the startcodon can be suitably chosen from GTG or TTG. The multicistronic transcription units in this aspect are at least tricistronic.

The practice of this invention will employ, unless otherwise indicated, conventional techniques of immunology, molecular biology, microbiology, cell biology, and recombinant DNA, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

The invention is further explained in the following examples. The examples do not limit the invention in any way. They merely serves to clarify the invention.

### Examples

Example 1 describes the selection system with the multicistronic transcription unit of the present invention, and it will be clear that the variations described in examples 8-26 of WO 2006/048459, can also be applied and tested for the multicistronic transcription units of the present application. The same holds for those of examples 20-27 of US 2006/0195935.

### Example 1: Stringent selection by placing a modified Zeocin resistance gene behind an IRES sequence

Examples 8-26 of WO 2006/048459 have shown a selection system where a sequence encoding a selectable marker protein is upstream of a sequence encoding a protein of interest in a multicistonic transcription unit, and wherein the translation initiation sequence of the selectable marker is non-optimal, and wherein further internal ATGs have been removed from the selectable marker coding sequence. This system results in a high stringency selection system. For instance the Zeo selection marker wherein the translation initiation codon is changed into TTG was shown to give very high selection stringency, and very high levels of expression of the protein of interest encoded downstream.

In another possible selection system (i.e. the system of the present invention) the selection marker, e.g. Zeo, is placed downstream from an IRES sequence. This creates a multicistronic mRNA from which the Zeo gene product is translated by IRES-dependent initiation. In the usual d2EGFP-IRES-Zeo construct (i.e. a construct of the prior art, e.g. WO 2006/005718), the Zeo startcodon is the optimal ATG. We tested whether changing the Zeo ATG startcodon into for instance TTG (referred to as IRES-TTG Zeo) resuls in increased selection stringencies compared to the usual IRES-ATG Zeo.

### Results

The used constructs are schematically shown in FIG. 1. The control construct consisted of a CMV promoter, the d2EGFP gene, an IRES sequence (the sequence of the used IRES (Rees et al, 1996) in this example was:
GCCCCTCTCCCTCCCCCCCCCCTAACGTTACTGGCCGAAGCCGCTTGGAATAAGGCC GGTGTGCGTTTGTCTATATGTGATTTTCCACCATATTGCCGTCTTTTGGCAATGTGAG GGCCCGGAAACCTGGCCCTGTCTTCTTGACGAGCATTCCTAGGGGTCTTTCCCCTCTC GCCAAAGGAATGCAAGGTCTGTTGAATGTCGTGAAGGAAGCAGTTCCTCTGGAAGC TTCTTGAAGACAAACAACGTCTGTAGCGACCCTTTGCAGGCAGCGGAACCCCCCACC TGGCGACAGGTGCCTCTGCGGCCAAAAGCCACGTGTATAAGATACACCTGCAAAGG CGGCACAACCCCAGTGCCACGTTGTGAGTTGGATAGTTGTGGAAAGAGTCAAATGG CTCTCCTCAAGCGTATTCAACAAGGGGCTGAAGGATGCCCAGAAGGTACCCCATTGT ATGGGATCTGATCTGGGGCCTCGGTGCACATGCTTTACATGTGTTTAGTCGAGGTTA AAAAAACGTCTAGGCCCCCCGAACCACGGGGACGTGGTTTTCCTTTGAAAAACACG ATGATAAGCTTGCCACAACCCCGGGATA; SEQ. ID. NO. 82), and a TTG Zeo selection, marker, i.e. the zeocin resistance gene with a TTG startcodon ('d2EGFP-IRES-TTG Zeo'). The other construct was the same, but with a combination of STAR 7 and STAR 67 placed upstream of the expression cassette and STAR 7 downstream of the cassette ('STAR7/67 d2EGFP-IRES-TTG Zeo STAR7'). Both constructs were transfected to CHO-K1 cells and selection was performed with 100 µg/ml Zeocin in the culture medium. Four colonies emerged after transfection with the control construct and six with the STAR containing construct. These independent colonies were isolated propagated before analysis of d2EGFP expression levels. As shown in FIG. 1, incorporation of STAR elements in the construct resulted in the formation of colonies with high d2EGFP expression levels. Of the control colonies without STAR elements ('d2EGFP-IRES-TTG Zeo') only one colony displayed some d2EGFP expression. The expression levels are also much higher than those obtained with other control constructs, containing the IRES with a normal Zeo with standard ATG startcodon, either with or without STAR elements ('d2EGFP-IRES-ATG Zeo' and 'STAR 7/67 d2EGFP-IRES-ATG Zeo STAR7'; also in these ATG Zeo constructs there was an enhancing effect of the STAR elements, but these are modest as compared to the novel TTG Zeo variant).

These results show that placing a Zeo selection marker with a TTG startcodon downstream of an IRES sequence, in combination with STAR elements, operates well and establishes a stringent selection system.

From these data and examples 8-26 of WO 2006/048459 and 20-27 of US 2006/0195935, it will be clear that the marker can be varied along the same lines of examples 8-26 of WO 2006/048459 and 20-27 of US 2006/0195935. For instance, instead of a TTG startcodon, a GTG startcodon can be used, and the marker can be changed from Zeo into a different, marker, e.g. Neo, Blas, dhfr, puro, etc, all with either GTG or TTG as startcodon. The STAR elements can be varied by using different STAR sequences or different placement thereof, or by substituting them for other chromatin control elements, e.g. MAR sequences. This leads to improvements over the prior art selection systems having an IRES with a marker with a normal ATG startcodon.

As a non-limiting example, instead of the modified Zeo resistance gene (TTG Zeo) a modified Neomycin resistance gene is placed downstream of an IRES sequence. The modification consists of a replacement of the ATG translation initiation codon of the Neo coding sequence by a TTG translation initiation codon, creating TTG Neo. The CMV-d2EGF-IRES-TTG Neo construct, either surrounded by STAR elements or not, is transfected to CHO-K1 cells. Colonies are picked, cells are propagated and d2EGFP values are measured. This ('IRES-TTG Neo') leads to improvement over the known selection system having Neo with an ATG startcodon downstream of an IRES ('IRES-ATG Neo'). The improvement is especially apparent when the TTG Neo construct comprises STAR elements.

**Example 2: Stability of expression by placing a modified dhfr gene behind an IRES sequence**

Modification of the translation initation codon of the Zeocin selection marker to a translation initiation codon that is used much less frequently than the usual ATG codon, results in a high stringency selection system. In the described selection system of WO 2006/048459, the TTG Zeo is placed upstream of the gene of interest. In another possible selection system the Zeo selection marker was placed downstream of an IRES sequence (present application, see example 1). This creates a bicistronic mRNA from which the Zeo gene product is translated from translation initiation codons in the IRES sequence.

In this experiment we combined embodiments of these two systems. We placed a TTG selection marker upstream of the reporter gene and coupled a GTG or TTG modified metabolic marker with an IRES to the reporter gene. Different selection marker genes can be used, such as the Zeocin and neomycin resistance genes, as well as the dhfr gene. Here we placed a modified Zeocin resistance gene, TTG Zeo (see WO 2006/048459), upstream of a gene of interest and the dhfr selection gene downstream of the gene of interest, coupled by an IRES (Fig. 2). The objective of this expression cassette was to select a mammalian cell clone producing high level of protein, first by selection on Zeocin. The TTG Zeo-gene of interest configuration most effectively achieves this objective. After this initial selection phase, the characteristics of the dhfr-protein are employed to achieve maintenance of the high expression levels in the absence of the Zeocin antibiotic.

Active selection pressure appears beneficial to keep the protein expression levels in a TTG Zeo selected colony at the same high level over a prolonged period of time. This can for instance be accomplished by keeping a minimal amount of Zeocin in the culture medium, but this is not favoured in industrial settings for economic and potentially for regulatory purposes (Zeocin is both toxic and expensive).

Another approach is to couple the gene of interest to a selection marker that is an enzyme that metabolizes one or more essential steps in a metabolic pathway. With essential is meant that the cell is not able to synthesize specific essential metabolic building blocks itself, implying that these building blocks have to be present in the culture medium in order to allow the cell to survive. Well-known examples are the essential amino acids that cannot be synthesized by a mammalian cell and that need to be present in the culture medium to allow the cell to survive. Another example is related to the 5,6,7,8-tetrahydrofolate synthesizing *dhfr* gene. The corresponding dhfr protein is an enzyme in the folate pathway. The dhfr protein specifically converts folate into 5,6,7,8-tetrahydrofolate, a methyl group shuttle required for the de novo synthesis of purines (Hypoxanthine), thymidylic acid (Thymidine), and the amino acid Glycine. To operate, the non-toxic substance folate has to be present in the culture medium (Urlaub et al, 1980). Furthermore, the medium has to lack hypoxanthine and thymidine, since when these are available for the cell, the need for the dhfr enzyme is bypassed. CHO-DG44 cells lack the *dhfr* gene and these cells therefore need glycine, hypoxanthine and thymidine in the culture medium to survive. If, however, the end-products glycine, hypoxanthine and thymidine are absent from the culture medium and folate is present, and the *dhfr* gene is provided because it is present on an expression cassette in the cell, the cell can convert folate into 5,6,7,8-tetrahydrofolate, and can thus survive in this culture medium. This principle has been used for many years as selection methodology to create stably transfected mammalian cell lines.

Here, we use this principle, not to select the stable clones initially (this is done with Zeocin), but to keep the cells under metabolic selection pressure. The advantage is that initial very high protein expression can be achieved through the TTG Zeo selection system, and that these high expression levels can be maintained, without the need to keep Zeocin in the culture medium. Instead, Zeocin can be omitted from the medium and the absence of Glycin, Hypoxantine and Thymidine (GHT) or just Hypoxantine and Thymidine (HT) from the culture medium is sufficient to keep the selection pressure high enough to warrant high protein expression levels. Such a configuration requires the presence of two selection markers, both the Zeocin resistance gene and the *dhfr* gene need to present on the expression cassette. As outlined above this is efficiently achieved when both genes are present with the gene of interest in such a configuration that a tricistronic mRNA is transcribed form a single promoter. When the modified Zeocin resistance gene (TTG Zeo) is employed upstream of the d2EGFP gene, the *dhfr* gene needs to be downstream coupled to the d2EGFP gene, for instance through an IRES sequence (Fig. 1).

### Results

We made constructs in which the TTG Zeo selection marker was placed upstream of the d2EGFP reporter gene and the dhfr selection marker downstream of the d2EGFP gene, coupled through an IRES sequence (Fig. 2). These constructs were flanked with STARs 7/67/7. Three versions of these constructs were made: ATG dhfr, GTG dhfr or TTG dhfr, each name indicating the startcodon used for the *dhfr* gene. The constructs were transfected to CHO-DG44 cells. DNA was transfected using Lipofectamine 2000 (Invitrogen) and cells were grown in the presence of 400 µg/ml Zeocin in IMDM medium (Gibco) + 10% FBS (Gibco) + HT-supplement.

The average d2EGFP value in 14 TTG Zeo IRES **ATG** dhfr clones was 341 (day 1), when measured in the presence of 400 µg/ml Zeocin (Fig. 2). After these measurements the cells were split and further cultured under three conditions:
(1) with 400 µg/ml Zeocin and with hypoxanthine and thymidine (HT-supplement) in the medium,
(2) without Zeocin, but with HT-supplement in the medium,
(3) without Zeocin and without HT-supplement.

In summary, in condition 1, the cells are under Zeocin selection pressure only, in condition 2 the cells are NOT under any selection pressure and in condition 3 the cells remain under DHFR selection pressure. The latter condition 3 requires continuous expression of the dhfr gene to allow expression of the dhfr protein and cell survival as a result.

After 65 days we again measured the d2EGFP values. The average d2EGFP value in the TTG Zeo IRES **ATG** dhfr clones under Zeocin selection was now 159 (Fig. 2). The average d2EGFP value in the TTG Zeo IRES ATG dhfr clones without Zeocin and with HT supplement was 20 (Fig. 2). The average d2EGFP value in the TTG Zeo IRES ATG dhfr clones without Zeocin selection and without HT supplement was 37 (Fig: 2). Overall we thus observed a drop in d2EGFP values, but the most severe in the absence of Zeocin, irrespective whether HT supplement was present or not.

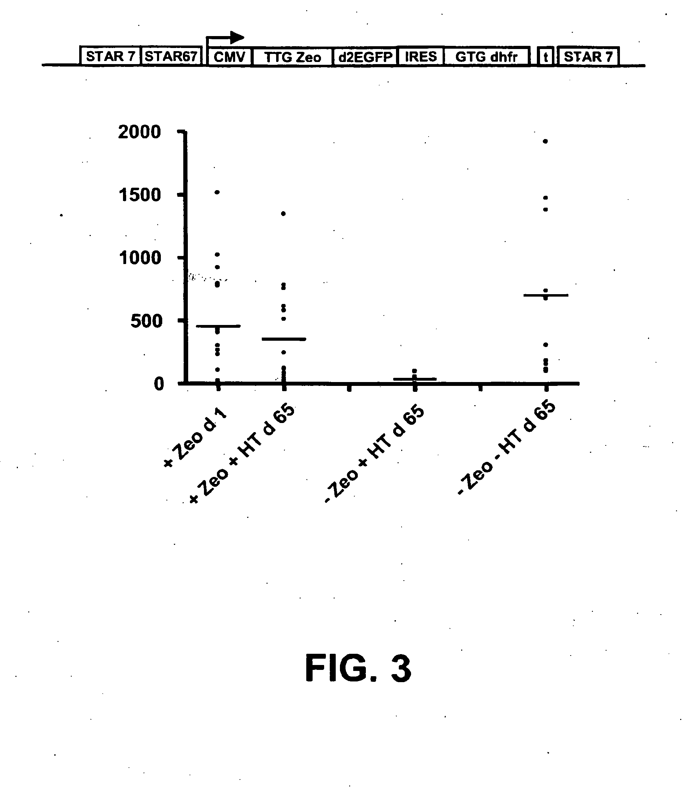

We followed the same protocol with the TTG Zeo IRES **GTG** dhfr construct. The average d2EGFP value in 15 TTG Zeo IRES GTG dhfr clones was 455 (day 1), when measured in the presence of 400 µg/ml Zeocin (Fig. 3). After these measurements the cells were split and further cultured under the above described three conditions. After 65 days we again measured the d2EGFP values. The average d2EGFP value in the TTG Zeo IRES GTG dhfr clones under Zeocin selection was now 356 (Fig. 3). The average d2EGFP value in the TTG Zeo IRES GTG dhfr clones without Zeocin selection and with HT supplement was 39 (Fig. 3). The average d2EGFP value in the TTG Zeo IRES GTG dhfr clones without Zeocin selection and without HT supplement was 705 (Fig. 3).

In this case we thus observed a drop in d2EGFP values only in the absence of Zeocin and in the presence of HT supplement (condition 2). In the absence of Zeocin, but in the absence of also HT supplement the d2EGFP values became actually significantly higher (condition 3). This may indicate that the expression levels of the dhfr protein, due to the impaired translation frequency of the GTG dhfr mRNA is low enough to provide very high selection stringency. This selection pressure, in the absence of any toxic agents, is high enough to maintain high protein expression levels over time, and apparently even improves these expression levels over time.

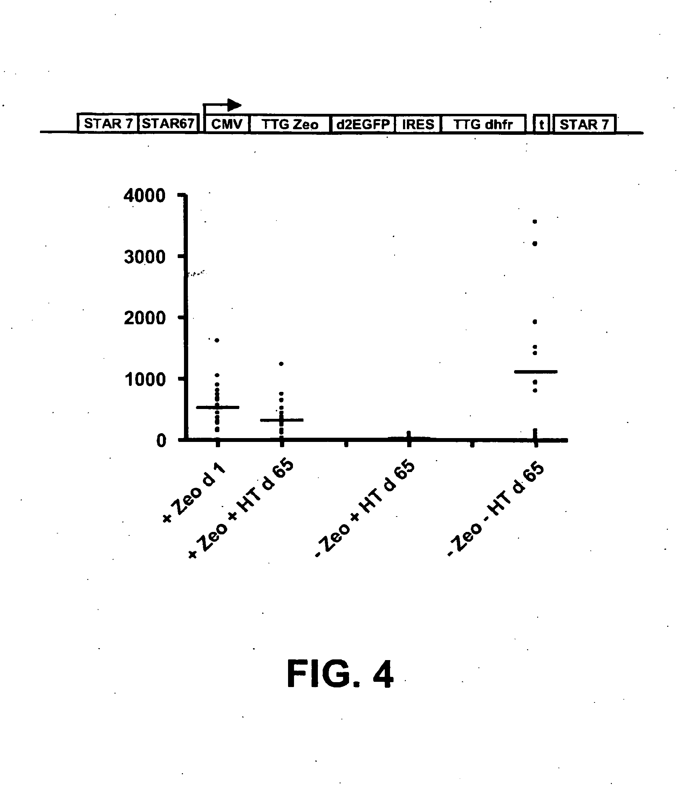

We did the same for the TTG Zeo IRES **TTG** dhfr construct. The average d2EGFP value in 18 TTG Zeo IRES TTG dhfr clones was 531 (day 1), when measured in the presence of 400 µg/ml Zeocin (Fig. 4). After these measurements the cells were split and further cultured under the above described three conditions. After 65 days we again measured the d2EGFP values. The average d2EGFP value in the TTG Zeo IRES TTG dhfr clones under Zeocin selection was now 324 (Fig. 4). The average d2EGFP value in the TTG Zeo IRES TTG dhfr clones without Zeocin selection and in the presence of HT supplement was 33 (Fig. 4). The average d2EGFP value in the TTG Zeo IRES TTG dhfr clones without Zeocin selection and without HT supplement was 1124 (Fig. 4).

Again, we observed a drop in d2EGFP values only in the absence of Zeocin and in the presence of HT supplement (condition 2). In the absence of Zeocin, but in the absence of HT supplement the d2EGFP values became even higher than with the TTG Zeo IRES GTG dhfr construct (condition 3). Since the TTG variant is more stringent than the GTG variant, it is expected that even less dhfr protein will be translated with the TTG dhfr than with the GTG dhfr variant. The increased selection pressure, in the absence of any toxic agents, with the TTG dhfr variant is high enough to maintain high protein expression levels over time, and apparently also even further improves protein expression levels over time.

The data show that coupling a non-ATG startcodon-variant of the *dhfr* gene through an IRES to the d2EGFP gene allows a high degree of stability of high d2EGFP expression in CHO-DG44 cells. This occurs in culture medium without Zeocin and without essential metabolic end products. Prior selection on Zeocin through the modified TTG Zeo selection marker allows the efficient establishment of colonies with high d2EGFP expression levels. Now just a simple change of culture medium (removing Zeocin and HT) is required to maintain the high d2EGFP expression levels, and even improve these expression levels.

### Example 3: Increased expression by placing a modified dhfr gene behind a weakened IRES sequence is not the result of gene amplification

Use of the *dhfr* gene as a selection marker in the prior art often relied on amplification of the *dhfr* gene. A toxic agent, methotrexate was used in such systems to amplify the dhfr gene, and concomitantly therewith the desired transgene, of which up to many thousands of copies could be found integrated into the genome of CHO cells after such amplification. Although these high copy numbers lead to high expression levels, they are also considered a disadvantage because so many copies can lead to increased genomic instability, and further removal of methotrexate from the culture medium leads to rapid removal of many of the amplified loci.

In example 2, no methotrexate was used to inhibit the dhfr enzyme activity. Only the hypoxanthine and thymidine precursor were removed from the culture medium, and this was sufficient to achieve both stability of protein expression, and even increased expression levels. We therefore determined whether the employment of the dhfr enzyme in our setting resulted in gene amplification.

### Results

We isolated DNA from the clones that were described in Example 2, on the same day (65) that the d2EGFP values were measured. With this DNA we determined the d2EGFP copy numbers.

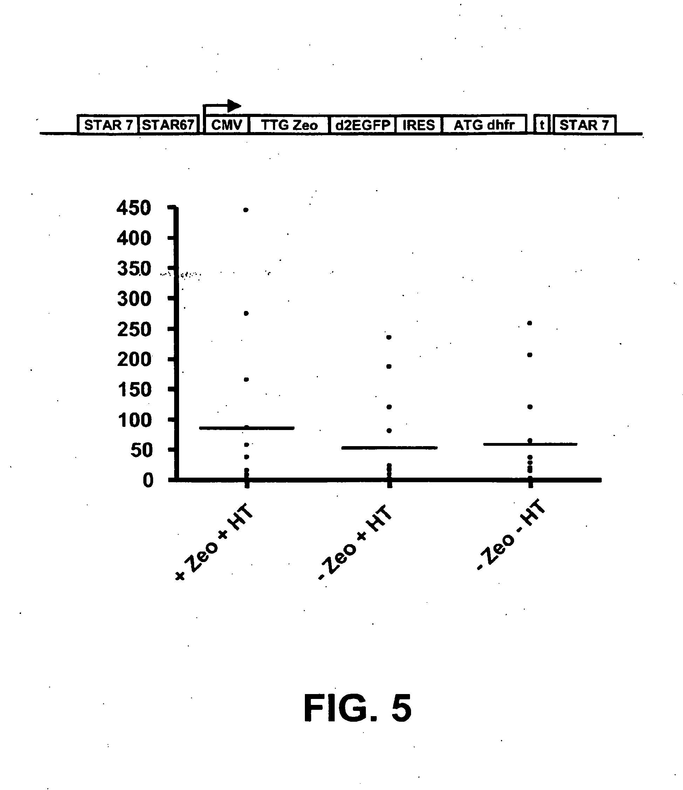

The average d2EGFP copy number in the TTG Zeo IRES **ATG** dhfr clones under Zeocin selection was 86 (condition 1)(Fig. 5). The average d2EGFP copy number in the TTG Zeo IRES ATG dhfr clones without Zeocin selection and in the presence of HT supplement was 53 (condition 2)(Fig. 5). The average d2EGFP copy number in the TTG Zeo IRES ATG dhfr clones without Zeocin selection and without HT supplement was 59 (condition 3)(Fig. 5).

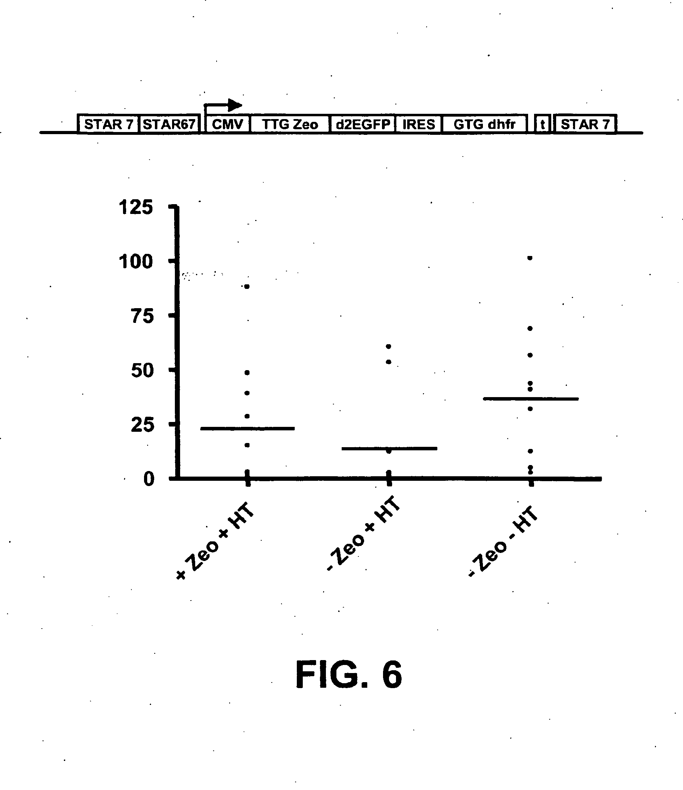

The average d2EGFP copy number in the TTG Zeo IRES **GTG** dhfr clones under Zeocin selection was 23 (condition 1)(Fig. 6). The average d2EGFP copy number in the TTG Zeo IRES GTG dhfr clones without Zeocin selection and in the presence of HT supplement was 14 (condition 2)(Fig. 6). The average d2EGFP copy number in the TTG Zeo IRES GTG dhfr clones without Zeocin selection and without HT supplement was 37 (condition 3)(Fig. 6).

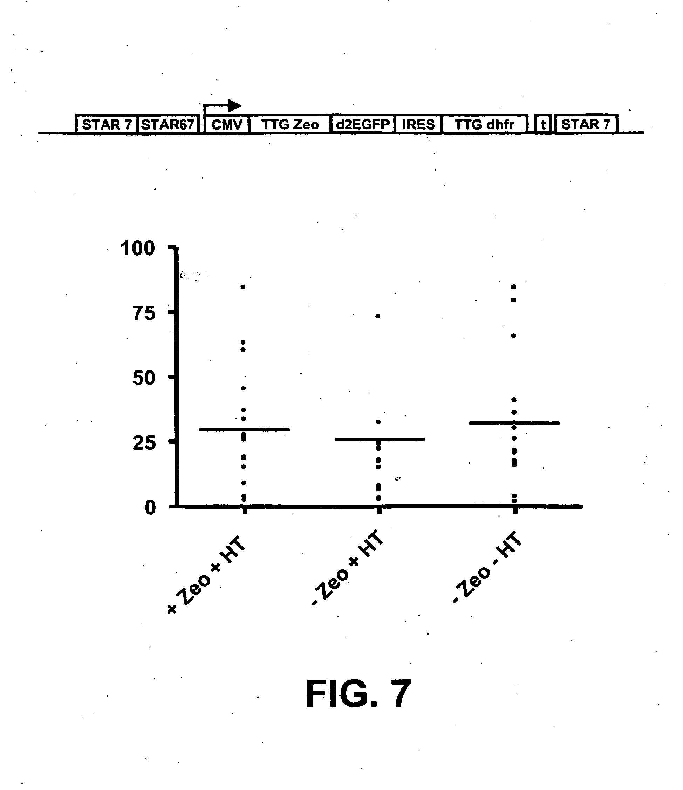

The average d2EGFP copy number in the TTG Zeo IRES **TTG** dhfr clones under Zeocin selection was 33 (condition 1)(Fig. 7). The average d2EGFP copy number in the TTG Zeo IRES TTG dhfr clones without Zeocin selection and in the presence of HT supplement was 26 (condition 2)(Fig. 7). The average d2EGFP copy number in the TTG Zeo IRES TTG dhfr clones without Zeocin selection and without.HT supplement was 32 (condition 3)(Fig. 7).

In neither case we observed a strong increase of the d2EGFP copy numbers after removal of HT supplement, which resulted in the increased d2EGFP values in case of the GTG dhfr and TTG dhfr variant. The fact that with both constructs the d2EGFP values remained stable over time and even increased significantly must be due to the action of the dhfr protein. Still, no increased d2EGFP copy numbers were observed in the TTG Zeo TTG dhfr clones at all, and only a modest increase in the TTG Zeo GTG dhfr clones. Interestingly, the overall d2EGFP copy numbers in the lowest producers, the TTG Zeo **ATG** dhfr clones were higher than in the other two variants, while these clones did not maintain the initial high d2EGFP fluorescence values (see Example 2). We conclude from these data that the commonly known gene amplification, observed when using the dhfr protein in combination with the addition of methotrexate, is not responsible for keeping the d2EGFP expression levels stable over time and for the observed increase in these expression levels. Instead, it appears that per d2EGFP gene copy more d2EGFP protein is expressed with the GTG and TTG dhfr variants.

We have further analysed the d2EGFP mRNA levels for the different clones and under the different conditions as above, and found that these mRNA levels broadly followed the trend of the d2EGFP fluorescence values. We therefore conclude that the increases in the d2EGFP fluorescence values are due to increased mRNA levels, and not to altered translation efficiencies.

### References

Kaufman, RJ. (2000) Overview of vector design for mammalian gene expression Mol Biotechnol 16, 151-160.
Kozak M. (1986) Point mutations define a sequence flanking the AUG initiator codon that modulates translation by eukaryotic ribosomes. Cell 44: 283-292.
Kozak M. (1987) An analysis of 5'-noncoding sequences from 699 vertebrate messenger RNAs. Nucleic Acids Res. 15: 8125-8148.
Kozak M. (1989) Context effects and inefficient initiation at non-AUG codons in eucaryotic cell-free translation systems. Mol Cell Biol. 9: 5073-5080.
Kozak M. (1990) Downstream secondary structure facilitates recognition of initiator codons by eukaryotic ribosomes. Proc Natl Acad Sci USA 87:8301-8305.
Kozak M. (1997) Recognition of AUG and alternative initiator codons is augmented by G in position +4 but is not generally affected by the nucleotides in positions +5 and +6. EMBO J. 16: 2482-2492.
Kozak M. (2002) Pushing the limits of the scanning mechanism for initiation of translation. Gene 299: 1-34.
Lopez de Quinto, S, and Martinez-Salas, E. (1998) Parameters influencing translational efficiency in aphthovirus IRES- based bicistronic expression vectors Gene 217, 51-6.
Martinez-Salas, E. (1999) Internal ribosome entry site biology and its use in expression vectors Curr Opin Biotechnol 10, 458-64.
McBurney, MW, Mai, T, Yang, X, and Jardine, K. (2002) Evidence for repeat-induced gene silencing in cultured Mammalian cells: inactivation of tandem repeats of transfected genes Exp Cell Res 274, 1-8.
Mizuguchi, H, Xu, Z, Ishii-Watabe, A, Uchida, E, and Hayakawa, T. (2000) IRES-dependent second gene expression is significantly lower than cap- dependent first gene expression in a bicistronic vector Mol Ther 1, 376-82.
Rees, S, Coote, J, Stables, J, Goodson, S, Harris, S, and Lee, MG. (1996) Bicistronic vector for the creation of stable mammalian cell lines that predisposes all antibiotic-resistant cells to express recombinant protein Biotechniques 20, 102-104, 106, 108-110.
Urlaub, G. & Chasin, L.A. Isolation of Chinese hamster cell mutants deficient in dihydrofolate reductase activity. Proc Natl Acad Sci USA 77,4216-20 (1980)
Venkatesan, A, and Dasgupta, A. (2001) Novel fluorescence-based screen to identify small synthetic internal ribosome entry site elements Mol Cell Biol 21, 2826-37.
Whitelaw, E, Sutherland, H, Kearns, M, Morgan, H, Weaving, L, and Garrick, D. (2001) Epigenetic effects on transgene expression Methods Mol Biol 158, 351-68.

## Claims

1. A DNA molecule comprising: a multicistronic transcription unit comprising at least one coding sequence coding for both
i) a polypeptide of interest, and
ii) a selectable marker polypeptide functional in a eukaryotic host cell,
wherein the polypeptide of interest has a translation initiation sequence separate from that of the selectable marker polypeptide,
wherein the at least one coding sequence for the polypeptide of interest is upstream from the at least one coding sequence for the selectable marker polypeptide in said multicistronic transcription unit,
wherein an internal ribosome entry site (IRES) is present downstream from the at least one coding sequence for the polypeptide of interest and upstream from the at least one coding sequence for the selectable marker polypeptide, and
**characterized in that** the coding sequence coding for the selectable marker polypeptide comprises a translation start sequence selected from the group consisting of:
a) a GTG start codon;
b) a TTG start codon;
c) a CTG start codon;
d) a ATT start codon; and
e) a ACG start codon.

2. A DNA molecule according to claim 1, wherein the translation start sequence for the selectable marker polypeptide comprises a GTG start codon or a TTG start codon.

3. A DNA molecule according to any one of the preceding claims, wherein the selectable marker polypeptide provides resistance against lethal or growth-inhibitory effects of a selection agent.

4. A DNA molecule according to claim 3, wherein said selection agent is selected from the group consisting of zeocin, puromycin, blasticidin, hygromycin, neomycin, methotrexate, methionine sulphoximine and kanamycin.

5. A DNA molecule according to claim 3, wherein the selection agent is zeocin.

6. A DNA molecule according to claim 1 or 2, wherein the selectable marker polypeptide is a 5,6,7,8-tetrahydrofolate synthesizing enzyme (dhfr).

7. A DNA molecule according to any one of the preceding claims, wherein the multicistronic transcription unit further comprises a sequence encoding a second selectable marker polypeptide functional in a eukaryotic cell, wherein said sequence encoding a second selectable marker polypeptide:
a) has a translation initiation sequence separate from that of the polypeptide of interest,
b) is positioned upstream of said sequence encoding a polypeptide of interest,
c) has no ATG sequence in the coding strand following the startcodon of said second selectable marker polypeptide up to the startcodon of the polypeptide of interest, and
d) has a GTG startcodon or a TTG startcodon.

8. An expression cassette comprising a DNA molecule according to any one of the peceding claims, said expression cassette comprising a promoter upstream of said multicistronic transcription unit and a transcription termination sequence downstream of the multicistronic transcription unit, wherein said expression cassette is functional in a eukaryotic host cell for initiating transcription of the multicistronic transcription unit.

9. An expression cassette according to claim 8, further comprising at least one chromatin control element selected from the group consisting of a matrix or scaffold attachment region (MAR/SAR), an insulator sequence, an universal chromatin opening element (UCOE), and an anti-repressor (STAR) sequence.

10. An expression cassette according to claim 9, wherein said at least one chromatin control element is an anti-repressor sequence selected from the group consisting of:
a) any one of SEQ. ID. NO. 1 through SEQ. ID. NO. 66;
b) fragments of any one of SEQ. ID. NO. 1 through SEQ. ID. NO. 66, wherein said fragments have anti-repressor activity;
c) sequences that are at least 70% identical in nucleotide sequence to a) or b) wherein said sequences have anti-repressor activity; and
d) the complement of any one of a) to c).

11. A host cell comprising a DNA molecule according to any one of claims 1-7 or an expression cassette according to any one of claims 8-10, the host cell preferably being a mammalian cell, preferably a Chinese hamster ovary (CHO) cell.

12. A method of generating a host cell able to express a polypeptide of interest, said method comprising the steps of:
a) introducing into a plurality of precursor cells a DNA molecule according to any one of claims 1-7 or an expression cassette according to any one of claims 8-10, and
b) culturing the plurality of precursor cells under conditions suitable for expression of the selectable marker polypeptide, and
c) selecting at least one host cell expressing the polypeptide of interest.

13. A method of expressing a polypeptide of interest, comprising culturing a host cell comprising an expression cassette according to any one of claims 8-10, and expressing the polypeptide of interest from the expression cassette.

14. A method according to claim 13, further comprising harvesting the polypeptide of interest.

15. A method according to claim 13 or 14, wherein said host cells are CHO cells that have a dhfr phenotype and wherein the expression cassette comprises a coding sequence for a selectable marker polypeptide that is a 5,6,7,8-tetrahydrofolate synthesizing enzyme (dhfr), wherein said cells are cultured in a culture medium that contains folate and which culture medium is essentially devoid of hypoxanthine and thymidine.

## Patentansprüche

1. DNA-Molekül, umfassend: eine multicistronische Transkriptionseinheit, umfassend mindestens eine codierende Sequenz, die sowohl
i) ein Polypeptid von Interesse
ii) als auch ein in einer eukaryontischen Wirtszelle funktionelles,
selektierbares Markerpolypeptid
codiert,
wobei das Polypeptid von Interesse eine Translations-Startsequenz hat, die von der des selektierbaren Markerpolypeptids getrennt ist,
wobei die mindestens eine codierende Sequenz für das Polypeptid von Interesse stromaufwärts der mindestens einen codierenden Sequenz für das selektierbare Markerpolypeptid in der multicistronischen Transkriptionseinheit ist,
wobei eine interne Ribosomeneintrittsstelle (IRES) stromabwärts der mindestens einen codierende Sequenz für das Polypeptid von Interesse und stromaufwärts der mindestens einen codierende Sequenz für das selektierbare Markerpolypeptid vorhanden ist, und
**dadurch gekennzeichnet, dass** die codierende Sequenz, die das selektierbare Markerpolypeptid codiert, eine Translations-Startsequenz umfasst, ausgewählt aus der Gruppe bestehend aus:
a) einem GTG-Startcodon;
b) einem TTG-Startcodon;
c) einem CTG-Startcodon;
d) einem ATT-Startcodon; und
e) einem ACG-Startcodon.

2. DNA-Molekül nach Anspruch 1, wobei die Translations-Startsequenz für das selektierbare Markerpolypeptid ein GTG-Startcodon oder ein TTG-Startcodon umfasst.

3. DNA-Molekül nach einem der vorhergehenden Ansprüche, wobei das selektierbare Markerpolypeptid Resistenz gegenüber tödlichen oder wachstumshemmenden Wirkungen eines Selektionsagens gewährt.

4. DNA-Molekül nach Anspruch 3, wobei das Selektionsagens ausgewählt ist aus der Gruppe bestehend aus Zeocin, Puromycin, Blasticidin, Hygromycin, Neomycin, Methotrexat, Methioninsulphoximin und Kamanycin.

5. DNA-Molekül nach Anspruch 3, wobei das Selektionsagens Zeocin ist.

6. DNA-Molekül nach Anspruch 1 oder 2, wobei das selektierbare Markerpolypeptid ein 5,6,7,8-Tetrahydrofolat-synthetisierendes Enzym (dhfr) ist.

7. DNA-Molekül nach einem der vorhergehenden Ansprüche, wobei die multicistronische Transkriptionseinheit des Weiteren eine Sequenz umfasst, die ein zweites in einer eukaryontischen Zelle funktionelles Markerpolypeptid codiert, wobei die ein zweites selektierbares Markerpolypeptid codierende Sequenz:
a) eine Translations-Startsequenz aufweist, die von der des Polypeptids von Interesse getrennt ist,
b) stromaufwärts der Sequenz gelegen ist, die ein Polypeptid von Interesse codiert,
c) keine ATG-Sequenz in dem codierenden Strang aufweist, der auf das Startcodon des zweiten selektierbaren Markerpolypeptids folgt, bis zu dem Startcodon des Polypeptids von Interesse, und
d) ein GTG-Startcodon oder ein TTG-Startcodon aufweist.

8. Expressionskassette, die ein DNA-Molekül nach einem der vorhergehenden Ansprüche umfasst, wobei die Expressionskassette einen Promotor stromaufwärts
der
multicistronischen Transkriptionseinheit und eine Transkriptions-Terminationssequenz stromabwärts der multicistronischen Transkriptionseinheit umfasst, wobei die Expressionskassette in einer eukaryontischen Wirtszelle für den Start der Transkription der multicistronischen Transkriptionseinheit funktionell ist.

9. Expressionskassette nach Anspruch 8, des Weiteren umfassend mindestens ein Chromatin-Kontrollelement ausgewählt aus der Gruppe bestehend aus einem Matrix- oder Geriast-Anheftungsbereich (MAR/SAR), einer Isolatorsequenz, einem universalem Chromatin-Öffnungselement (UCOE) und einer Anti-Repressor (STAR)-Sequenz.

10. Expressionskassette nach Anspruch 9, wobei das mindestens eine Chromatin-Kontrollelement eine Anti-Repressor-Sequenz ist, ausgewählt aus der Gruppe bestehend aus:
a) einer beliebigen von SEQ ID NO:1 bis SEQ ID NO:66;
b) Fragmenten einer beliebigen von SEQ ID NO:1 bis SEQ ID NO:66, wobei die Fragmente Anti-Repressor-Aktivität aufweisen;
c) Sequenzen, die in der Nucleotidsequenz zu mindestens 70% identisch zu a) oder b) sind, wobei die Sequenzen Anti-Repressor-Aktivität aufweisen; und
d) das Komplement zu einem beliebigen von a) bis c).

11. Wirtszelle, die ein DNA-Molekül nach einem der Ansprüche 1 bis 7 oder eine Expressionskassette nach einem der Ansprüche 8 bis 10 umfasst, wobei die Wirtszelle bevorzugt eine Säugerzelle, vorzugsweise eine Ovarzelle des chinesischen Hamsters (CHO) ist.

12. Verfahren zur Generierung einer Wirtszelle, die zur Expression eines Polypeptids von Interesse fähig ist, umfassend die Schritte:
a) Einführen eines DNA-Moleküls nach einem der Ansprüche 1 bis 7 oder einer Expressionskassette nach einem der Ansprüche 8 bis 10 in einer Vielzahl von Vorläuferzellen,
b) Züchten der Vielzahl von Vorläuferzellen unter Bedingungen, die für die Expression des selektierbaren Markerpolypeptids geeignet sind, und
c) Auswählen mindestens einer Wirtszelle, die das Polypeptid von Interesse exprimiert.

13. Verfahren zur Expression eines Polypeptids von Interesse, umfassend das Züchten einer Wirtszelle, die eine Expressionskassette nach einem der Ansprüche 8 bis 10 umfasst, und Exprimieren des Polypeptids von Interesse aus der Expressionskassette.

14. Verfahren nach Anspruch 13, des Weiteren umfassend das Gewinnen des Polypeptids von Interesse.

15. Verfahren nach Anspruch 13 oder 14, wobei die Wirtszellen CHO-Zellen sind, die einen dhfr-Phänotyp aufweisen, und wobei die Expressionskassette eine codierende Sequenz aufweist, die ein selektierbares Markerpolypeptid codiert, das ein 5,6,7,8-Tetrahydrofolat-synthetisierendes Enzym (dhfr) ist, wobei die Zellen in einem Kulturmedium gezüchtet werden, das Folat enthält, und wobei das Kulturmedium im Wesentlichen frei von Hypoxanthin und Thymidin ist.

## Revendications

1. Molécule d'ADN comprenant: une unité de transcription polycistronique comprenant au moins une séquence codante codant à la fois pour:
i) un polypeptide d'intérêt ; et
ii) un polypeptide marqueur sélectionnable fonctionnel dans une cellule hôte eucaryote ;
dans laquelle le polypeptide d'intérêt possède une séquence d'initiation de la traduction séparée de celle du polypeptide marqueur sélectionnable ;
dans laquelle ladite au moins une séquence codante pour le polypeptide d'intérêt est située en amont de ladite au moins une séquence codante pour le polypeptide marqueur sélectionnable dans ladite unité de transcription polycistronique ;
dans laquelle un site d'entrée interne pour le ribosome (IRES) est présent en aval de ladite au moins une séquence codante pour le polypeptide d'intérêt et en amont de ladite au moins une séquence codante pour le polypeptide marqueur sélectionnable ; et
**caractérisée en ce que** la séquence codante codant pour le polypeptide marqueur sélectionnable comprend une séquence d'initiation de la traduction, choisi parmi le groupe constitué par :
a) un codon d'initiation GTG ;
b) un codon d'initiation TTG ;
c) un codon d'initiation CTG ;
d) un codon d'initiation ATT ; et
e) un codon d'initiation ACG.

2. Molécule d'ADN selon la revendication 1, dans laquelle la séquence d'initiation de la traduction pour le polypeptide marqueur sélectionnable comprend un codon d'initiation GTG ou un codon d'initiation TTG.

3. Molécule d'ADN selon l'une quelconque des revendications précédentes, dans laquelle le polypeptide marqueur sélectionnable confère une résistance aux effets létaux ou inhibiteurs de croissance d'un agent de sélection.

4. Molécule d'ADN selon la revendication 3, dans laquelle ledit agent de sélection est choisi parmi le groupe constitué par la zéocine, la puromycine, la blasticidine, l'hygromycine, la néomycine, le méthotrexate, la méthionine-suitoximine et la kanamycine.

5. Molécule d'ADN selon la revendication 3, dans laquelle l'agent de sélection est la zéocine.

6. Molécule d'ADN selon la revendication 1 ou 2, dans laquelle le polypeptide marqueur sélectionnable est une enzyme de synthèse du 5,6,7,8-tétrahydrofolate (dhfr).

7. Molécule d'ADN selon l'une quelconque des revendications précédentes, dans laquelle l'unité de transcription polycistronique comprend en outre une séquence codant pour un deuxième polypeptide marqueur sélectionnable fonctionnel dans une cellule eucaryote, dans laquelle ladite séquence codant pour un deuxième polypeptide marqueur sélectionnable :
a) possède une séquence d'initiation de la traduction séparée de celle du polypeptide d'intérét ;
b) est disposée en amont de ladite séquence codant pour un polypeptide d'intérèt ;
c) ne possède pas de séquence ATG dans le brin codant qui suit le codon d'initiation dudit deuxième polypeptide marqueur sélectionnable jusqu'au codon d'initiation du polypeptide d'intérêt ; et
d) possède un codon d'initiation GTG ou un codon d'initiation TTG.

8. Cassette d'expression comprenant une molécule d'ADN selon l'une quelconque des revendications précédentes, ladite cassette d'expression comprenant un promoteur en amont de ladite unité de transcription polycistronique et une séquence de terminaison de la transcription en aval de l'unité de transcription polycistronique, ladite cassette d'expression étant fonctionnelle dans une cellule hôte eucaryote pour déclencher la transcription de l'unité de transcription polycistronique.

9. Cassette d'expression selon la revendication 8, comprenant en outre au moins un élément de contrôle de la structure chromatinienne, choisi parmi le groupe constitué par une région de fixation sur la matrice ou sur la charpente (MAR/SAR), une séquence d'isolation, un élément ubiquitaire d'ouverture de la chromatine (UCOE) et une séquence antirépresseur (STAR).

10. Cassette d'expression selon la revendication 9, dans laquelle ledit au moins un élément de contrôle de la structure chromatinienne est une séquence antirépresseur choisie parmi le groupe constitué par :
a) l'une quelconque des séquences SEQ ID NO: 1 à SEQ ID NO: 66 ;
b) des fragments de l'une quelconque des séquences SEQ ID NO: 1 à SEQ ID NO: 66, ledit fragment possédant une activité d'antirépresseur ;
c) des séquences qui manifiestent une identité de séquence nucléotidique à concurrence d'au moins 70 % avec la séquence a) ou b), lesdites séquences possédant une activité d'antirépresseur ; et
d) le complément de l'un quelconque des éléments a) à c).

11. Cellule hôte comprenant une molécule d'ADN selon l'une quelconque des revendications 1 à 7 ou une cassette d'expression selon l'une quelconque des revendications 8 à 10, la cellule hôte représentant de préférence une cellule mammalienne, de préférence une cellule d'ovaire de hamster chinois (CHO).

12. Procédé de génération d'une cellule hôte capable d'exprimer un polypeptide d'intérêt, ledit procédé comprenant les étapes :
a) d'introduction, dans plusieurs cellules précurseurs, d'une molécule d'ADN selon l'une quelconque des revendications 1 à 7 ou d'une cassette d'expression selon l'une quelconque des revendications 8 à 10 ; et
b) de mise en culture desdites plusieurs cellules précurseurs dans des conditions appropriées pour l'expression du polypeptide marqueur sélectionnable ; et
c) de sélection d'au moins une cellule hôte exprimant le polypeptide d'intérêt.

13. Procédé d'expression d'un polypeptide d'intérêt, comprenant la mise en culture d'une cellule hôte comprenant une cassette d'expression selon l'une quelconque des revendications 8 à 10, et l'expression du polypeptide d'intérêt à partir de la cassette d'expression.

14. Procédé selon la revendication 13, comprenant en outre la récolte du polypeptide d'intérêt.

15. Procédé selon la revendication 13 ou 14, dans lequel lesdites cellules hôtes sont des cellules CHO qui possèdent un phénotype dhfr⁻, et dans lequel la cassette d'expression comprend une séquence codante pour un polypeptide marqueur sélectionnable qui représente une enzyme de synthèse du 5,6,7,8-tétrahydrofolate (dhfr), lesdites cellules étant cultivées dans un milieu de culture qui contient du folate et ledit milieu de culture étant essentiellement exempt d'hypoxanthine et de thymidine.
